# EUROPEAN PATENT SPECIFICATION

(11) **EP 1 833 752 B1**
(45) Date of publication and mention of the grant of the patent: **28.11.2018**
(21) Application number: 05783332.9
(22) Date of filing: 08.08.2005
(51) Int. Cl.: A61M 5/315, A61J 1/00, A61M 5/24, A61M 5/31

(54) **CONTAINER CLOSURE DELIVERY SYSTEM**
BEHÄLTERVERSCHLUSSZUFUHRSYSTEM
SYSTÈME DE DISTRIBUTION DE FERMETURES D'EMBALLAGES

(30) Priority: 30.12.2004 US 640625 P; 30.06.2005 US 172064
(43) Date of publication of application: 19.09.2007
(73) Proprietor: Integrity Biosolution, LLC, Camarillo, CA 93012 (US)
(72) Inventor: CHANG, Byeong S., Thousand Oaks, CA 91360 (US); LIU, Roger W., Moorpark, CA 93021 (US)
(74) Representative: Schlich, George
(86) International application number: PCT/US2005/028035
(87) International publication number: WO 2006/073505

(56) References cited:
- EP-A1- 0 298 585
- US-A- 3 739 947
- US-A- 3 766 917
- US-A- 3 766 917
- US-B1- 6 386 872
- US-B1- 6 386 872

## Description

### Technical Field

The field of the present invention is a container closure delivery system that is suitable for lyophilized pharmaceutical injectable products and which facilitates the easy, direct injection of the lyophilized product without the need for a reconstitution/mixing step of the powder and a liquid diluent.

### Background Art

Due to continued advances in genetic and cell engineering technologies, proteins known to exhibit various pharmacological actions in vivo are capable of production in large amounts for pharmaceutical applications. However, one of the most challenging tasks in the development of protein pharmaceuticals is to deal with the inherent physical and chemical instabilities of such proteins, especially in aqueous dosage forms. To try to understand and maximize the stability of protein pharmaceuticals and any other usable proteins, many studies have been conducted, especially in the past two decades. These studies have covered many areas, including protein folding and unfolding/denaturation, mechanisms of chemical and physical instabilities of proteins, as well as various means of stabilizing proteins in aqueous form; see, e.g., Manning et al., Pharm Res., 1989;6:903-918; Arakawa et al., Adv Drug Deliv Rev., 2001;46:307-326; Wang W., Int J Pharm., 1999;185:129-188; Chen T., Drug Dev Ind Pharm., 1992;18:1311-1354, and references cited therein.

Because of the instability issues associated with the aqueous dosage forms, powder formulations are generally preferred to achieve sufficient stability for the desired shelf life of the product. Various techniques to prepare dry powders have been known, substantiated and practiced in the pharmaceutical and biotech industry. Such techniques include lyophilization, spray-drying, spray-freeze drying, bulk crystallization, vacuum drying, and foam drying. Lyophilization (freeze-drying) is often a preferred method used to prepare dry powders (lyophilizates) containing proteins. Various methods of lyophilization are well known to those skilled in the art; see, e.g., Pikal MJ., In: Cleland JL, Langer R. eds. Formulation and Delivery of Proteins and Peptides. Washington, DC: American Chemical Society; 1994:120-133; Wang W., Int J Pharm. 2000;203:1-60, and references cited therein. The lyophilization process consists if three stages: freezing, primary drying, and secondary drying. Because the protein product is maintained frozen throughout drying process, lyophilization provides the following advantages over alternative techniques: minimum damage and loss of activity in delicate, heat-liable materials; speed and completeness of rehydration; the possibility of accurate, clean dosing into final product containers so that particulate and bacterial contamination is reduced; permits product reconstitution at a higher concentration than it was at the time of freezing; and permits storage of the product at ambient temperatures. The latter can be particularly useful for hospital products in areas that do not have ready access to freezers, especially ultra-cold freezers.

Unfortunately, even in solid dosage forms, some proteins can be relatively unstable and this instability may be a product of the lyophilization method used for preparing the solid dosage forms and/or the inherent instability of the actual solid dosage formulations themselves. For example, in certain instances, lyophilization processing events can force a protein to undergo significant chemical and physical changes. Such processing events include concentration of salts, precipitation, crystallization, chemical reactions, shear, pH, amount of residual moisture remaining after freeze-drying, and the like. Such chemical and physical changes include, e.g., formation of dimer or other higher order aggregates, and unfolding of tertiary structure. Unfortunately, these changes may result in loss of activity of the protein, or may result in significant portions of the active materials in the drug having been chemically transformed into a degradation product or products which may actually comprise an antagonist for the drug or which may give rise to adverse side effects. In addition to the instabilities incurred upon proteins because of the inherent steps of the lyophilization process, other disadvantages of lyophilization include: long and complex processing times; high energy costs; and expensive set up and maintenance of the lyophilization facilities. As such, use of lyophilization is usually restricted to delicate, heat-sensitive materials of high value. Additionally, lyophilized powders are typically formed as cakes, which require additional grinding and milling and optionally sieving processing steps to provide flowing powders. To try to understand and to optimize protein stability during lyophilization and after lyophilization, many studies have been conducted; see, e.g., Gomez G. et al., Pharm Res. 2001;18:90-97; Strambini GB., Gabellieri E., Biophys J., 1996;70:971-976; Chang BS. et al., J Pharm Sci., 1996;85:1325-1330, Pikal MJ., Biopharm, 1990;3:9, Izutsu K. et al., Pharm. Res., 1994;11-995, Overcashier DE., J Pharm Sci., 1999;88:688, Schmidt EA. et al., J Pharm Sci., 1999;88:291, and references cited therein.

In order to allow for parenteral administration of these powdered drugs, the drugs must first be placed in liquid form. To this end, the drugs are mixed or reconstituted with a diluent before being delivered parenterally to a patient. The reconstitution procedure must be performed under sterile conditions, and in some procedures for reconstituting, maintaining sterile conditions is difficult. One way of reconstituting a powdered drug is to inject a liquid diluent directly into a drug vial containing the powdered drug. This can be performed by use of a combination-syringe and syringe needle having diluent contained therein and drug vials which include a pierceable rubber stopper. The method of administration goes as follows: 1) the rubber stopper of the drug vial is pierced by the needle and the liquid in the syringe injected into the vial; 2) the vial is shaken to mix the powdered drug with the liquid; 3) after the liquid and drug are thoroughly mixed, a measured amount of the reconstituted drug is then drawn into the syringe; 4) the syringe is then withdrawn from the vial and the drug then be injected into the patient.

Other methods of administration of powdered drugs include the use of dual-chambered injection cartridges and/or pre-filled syringe systems. Injection cartridges of the dual-chamber type are well-known and have found a wide use. They are used together with various types of injection apparatuses which serve to hold the cartridge as it is readied for injection and as injections are subsequently administered. Injection cartridges of the dual-chamber type generally comprise a cylindrical barrel, which is shaped like a bottleneck at its front end and has an open rear end. The front end is closed by a septum of rubber or other suitable material, which is secured in place by means of a capsule. This capsule has a central opening where the septum is exposed and may be pierced by a hollow needle to establish a connection with the interior of the cartridge; see e.g., U.S Pat. No. 5,435,076 and references cited therein.

Dual-chambered pre-filled syringe systems are well known and have found wide commercial use; see e.g., U.S Pat. Nos. 5.080,649; 5,833,653; 6,419,656; 5,817,056; 5,489,266, and references cited therein. Pre-filled syringes of the dual-chambered type generally comprise an active ingredient which is lyophilized in one chamber, while a second chamber of the syringe contains a solvent that is mixed with the active substance immediately before application. In such devices, in order to facilitate the movement of the syringe plunger against compression of air, the chamber containing the lyophilized product typically has large head space and some additional mechanism, e.g., rotation of the plunger, screwing in the plunger, is necessary. As a result, the reconstituted drug needs to primed to remove large volumes of air prior to injection;
see e.g., U.S. Pat. No. 6,817,987 which describes a hypodermic syringe which holds a solvent and a soluble component (medicament) and wherein the solvent and medicament are mixed as the user presses and then releases the plunger of the syringe. Upon complete mixing, the user attaches a needle and then rotates the plunger of the syringe to allow for the injection.

Other devices used for reconstitution and delivery of powdered drugs are described in, e.g., U.S. Pat. Nos. 4,328,802; 4,410,321; 4,411,662; 4,432,755; 4,458,733; 4,898,209; 4,872,867; 3,826,260, and references cited therein. Unfortunately, all of these known methods require thorough reconstitution/mixing/priming of the lyophilized product into the diluent prior to injection and this reconstitution step can be complex, arduous and tedious for the patient. The need for this additional reconstitution/mixing/priming step renders injection of lyophilized product with convenient delivery devices such as autoinjectors unfeasible. On the contrary, liquid formulations do not require such preparation and can be delivered with convenient prefilled syringes and/or autoinjectors.

While these studies and advances have furthered the technology, there still clearly exists a need for improved storage stable powder drug formulations and improved lyophilization processes which are less complex and more economical, which do not lead to protein instability during processing, and which produce stable protein powders (at room temperature) for the desired shelf life of the product. There also still clearly exists a need for improved methods for the delivery of powdered drugs which do not require a reconstitution/mixing/priming step of the powdered drug with a diluent.

US 6 386 872 B1 discloses the features of the preamble of claim 1.

### Disclosure of Invention

According to a first aspect of the invention there is provided a delivery device for the administration of a powdered pharmaceutical product comprising the features of claim 1:
a first component having an ejection port at one end, a chamber holding a reservoir of diluent, and an activating means to facilitate the flow of said diluent through said ejection port; and
a second component containing a lyophilized drug powder, said second component capable of engaging said first component at one end and engaging a standard type needle at the opposing end, said second component comprising a one-way valve mechanism formed as a plunger assembly which upon activation of said first component, allows the diluent ejected from said first component to flow into said second component and encounter the powder in said second component, rapidly reconstitute and pass through said needle
characterised in that said second component is a container closure assembly comprising:
   a hard plug component comprising:
      a female luer slip fitting cavity to allow for friction fit of a standard type luer slip syringe;
      a circular cavity that can accommodate the standard type luer slip syringe; and
      a depression and female portion that can be mated with the sealing conical mound of the soft plug component to create a one way valve;
   a soft plug component comprising:
      a hollow inside capable of accepting said hard plug component to create the plunger assembly;
      vent holes which allow for vapors to escape during lyophilization processes;
      sealing ridges which serve to seal said soft plug component against the interior wall of the product container component; and
      a sealing conical mound that can be mated with said depression and female portion of said hard plug component to create a one way valve; and
   a product container component comprising:
      an open end having a chamber capable of holding a liquid to be lyophilized and capable of receiving said plunger assembly; and
      an opposing neck area having a detachable base to allow for attachment of a standard type luer slip or luer lock syringe needle, and
   in that there is no air space between the lyophilized drug powder and the plunger assembly.

According to a second aspect of the invention there is provided a process with the features of claim 2 for the preparation of a container closure assembly containing a lyophilized pharmaceutical powder product, for use in the first aspect, comprising the steps of:
1) loading an empty product container into an industry standard vial manufacturing filling line;
2) filling said product container with a liquid formulation containing a pharmaceutical product;
3) dropping a plunger assembly, the plunger assembly comprising a hard plug component inserted into a soft plug component, into the top of said product container to create a container closure assembly;
4) placing said container closure assembly into a lyophilizer apparatus;
5) subjecting said container closure assembly to a lyophilization process; and
6) completing said lyophilization process such that vertical compression of the lyophilizer shelves seals said plunger assembly into said product container to create a sealed container closure assembly containing lyophilized pharmaceutical powder product with minimal head space.

A container closure delivery system that is suitable for lyophilized pharmaceutical injectable products and facilitates the easy, direct injection of the lyophilized product without the need for a reconstitution/mixing step of the powder and a liquid diluent is disclosed. The present invention utilizes powder formulations and lyophilization processes that are optimized to produce powders which provide for "rapid" dissolution of the lyophilized powder, i.e., the powders are readily and immediately dissolved upon contact with a liquid diluent.

One object of the present invention is to provide a new container closure assembly suitable for lyophilized pharmaceutical injectable products and designed to provide for direct injection of a lyophilized product without the need for a reconstitution/mixing/priming step of the powder and diluent prior to injection. The container closure assembly of the present invention consists of three operating components designed to function in a manufacturing function and an end user function: a product container component; a soft plug component; and a luer slip/luer lock hard plug component. The container closure assembly is specifically designed to have minimal head space to avoid the need for priming. The product container component is specifically designed to hold a liquid to be lyophilized and capable of holding a plunger assembly. The soft plug component and hard plug component are specifically designed to engage with each other to form a plunger assembly with can then be inserted into the product container. Upon completion of the lyophilization process, the plunger assembly is compressed such that it rests directly on top of the powdered pharmaceutical product, i.e., there is no air space between the powder and the plunger assembly, and the plunger assembly serves as a one way valve to allow for the flow of liquid into the container closure assembly, i.e., allow for liquid to encounter the powder and rapidly reconstitute. Importantly, the container closure assembly is designed to utilize or be easily adaptable to industry standard or existing filling systems, providing a more economical alternative. Because of the unique assembly design, the container closure assembly facilitates the easy, direct injection of the lyophilized product without the need for a reconstitution/mixing/priming step of the powder and a liquid diluent by the end user.

Another object of the present invention is an improved process for the preparation of a container closure assembly containing a lyophilized powder product. This improved process comprises the following steps: 1) utilizing a industry standard vial manufacturing filling line, the product container is loaded into the equipment in a similar manner as regular vials; 2) the product container is filled with liquid active ingredient; 3) the hard plug portion is inserted snugly into the soft plug portion to create a plunger assembly; 4) the plunger assembly is dropped into an "open" position on top of the product container, sealing the product container in the same manner as lyophilization stoppers are mounted to regular vials; 5) the complete container closure assembly is then placed into the lyophilizer; 6) upon lyophilization, vapor is allowed to escape via the openings within the plunger assembly; and 7) upon completion of lyophilization, vertical compression of the lyophilizer shelves will seal the plunger assembly into the product container creating a sealed container closure assembly which retains the sterility of the active ingredient.

### Brief Description of Drawings

Figure 1 shows a cross-sectional view of the product container component of the container closure assembly of the present invention.
Figure 2 shows an isometric view of the soft plug portion of the container closure assembly of the present invention.
Figure 3 shows a cross sectional view of the soft plug portion of the container closure assembly of the present invention.
Figure 4 shows a cross sectional view of the hard plug portion of the container closure assembly of the present invention.
Figure 5 shows a cross sectional view of an embodiment of the container closure assembly whereupon a plunger assembly consisting of a soft plug portion and a hard plug portion are installed upon the product container after the filling the product container with liquid active ingredient and prior to placement of the container closure assembly within a freeze drying apparatus, i.e., the plunger assembly is installed in an "open" position in the product container.
Figure 6 shows a cross sectional area of an embodiment of the container closure assembly upon completion of the freeze drying cycle whereupon the liquid active ingredient has formed into a dry powder and the plunger assembly has been compressed by the freeze dryer shelves to create a sealed container closure assembly.
Figure 7 shows the intended use of the sealed container closure assembly of the present invention with a pre-filled syringe and needle. In Figure 7, the base attached to the neck area of the assembly has been broken off to allow for attachment of a needle.
Figure 8 is a graph depicting the 'gradient delivery' injection profile associated with the administration of a powdered drug using the powder formulations, lyophilization processes, and container closure assembly of the present invention. Protein concentration is plotted versus cumulative injection volume.
Figure 9 is a graph depicting an injection profile representative of those associated with the administration of powdered drugs using prior art devices which require a reconstitution and/or mixing step of the powdered drug with a diluent prior to injection. Protein concentration is plotted versus cumulative injection volume.

### Modes for Carrying Out the Invention

As those in the art will appreciate, the foregoing detailed description describes certain preferred embodiments of the invention in detail, and is thus only representative and does not depict the actual scope of the invention. Before describing the present invention in detail, it is understood that the invention is not limited to the particular aspects and embodiments described, as these may vary within the scope of the appended claims. It is also to be understood that the terminology used herein is for the purpose of describing particular embodiments only, and is not intended to limit the scope of the invention defined by the appended claims.

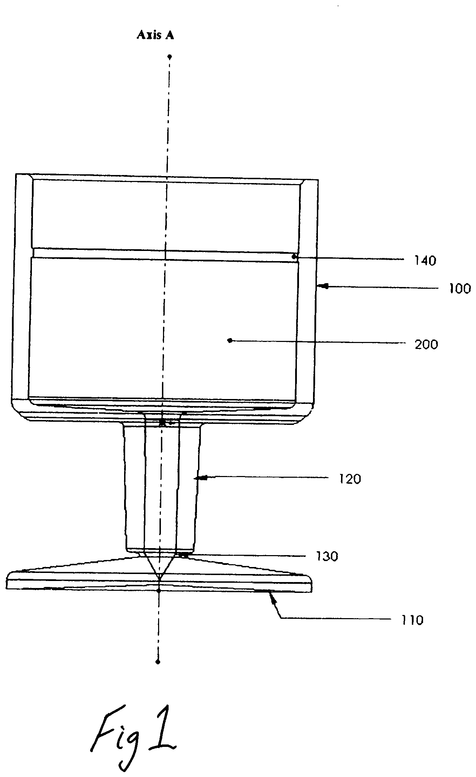

Referring now in more detail to the drawings, Figure 1 shows the product container **100** (also referred to herein as Component C) of the described container closure assembly **600.** The product container **100**, whose vertical axis is described by axis A, is constructed of a suitable plastic material, is cylindrical in shape, and has at one end an opening and at the other end an ejection port with detachable base **110.** The circular radius of the product container **100** wall creates a sufficient holding volume of liquid active ingredient **200.** Moving down the vertical axis A, the radius of the container reduces to form the neck area **120** of the product container. The outer surface area of this neck area **120** is of a sufficient radius to allow for a friction fit of a standard type luer slip or luer lock syringe needle attachment. At the end of the neck area **120**, a break or scoring point **130** is formed such that when the base **110** is torqued, it will break off at this point **130.** The base **110** is of a circular shape and designed to be grasped and torqued and removed when forces are presented in any matter other than vertically, along axis A. A locking ridge **140** is integrated into the sidewall of the product container **100** such that upon full insertion of the plunger assembly **500,** the plunger assembly **500** cannot be removed.

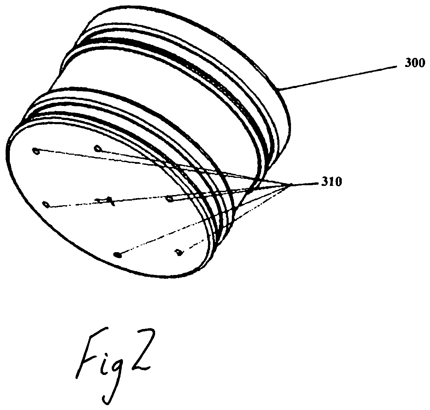

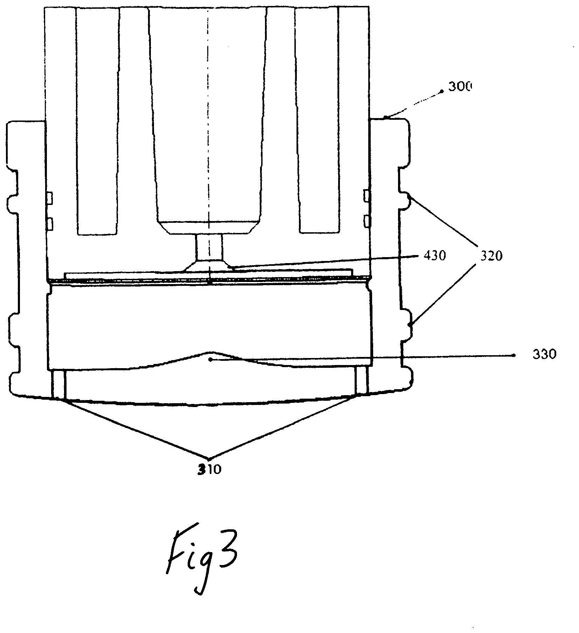

Figures 2 and 3 show the soft plug portion **300** (also referred to herein as Component B) of the described container closure assembly **600.** This soft plug portion **300** is envisaged to be constructed out of a suitable material that can offer appropriate sealing properties. The soft plug portion **300** has a hollow inside and is constructed to accept the hard plug portion **400** to create a plunger assembly **500** for the container closure assembly **600.** In Figure 2, vent holes **310** are depicted which allow for vapors to escape during lyophilization processes. In Figure 3, sealing ridges **320** are depicted which serve to seal the soft plug portion **300** against the interior wall of the product container **100.** Also depicted in Figure 3 is a sealing conical mound **330** which serves to seal the active ingredient during manufacturing and which is the male portion that when mated with the depression **430** of the hard plug portion **400** in the sealed container closure assembly **600,** will form a one way valve during patient use.

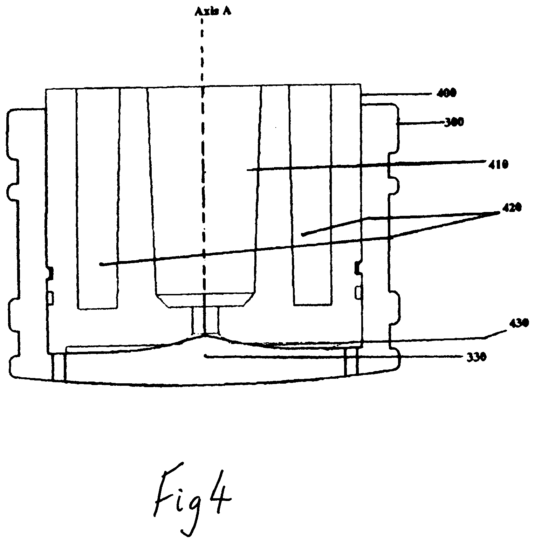

Figure 4 shows the luer slip/luer lock hard plug portion **400** (also referred to herein as Component A) of the described container closure assembly **600.** This hard plug portion **400,** whose vertical axis is described by axis A, is envisaged to be constructed of a suitable plastic material. In Figure 4, a female luer slip fitting cavity **410** is depicted where a standard type luer slip syringe can be frictionally attached. Also in Figure 4, a circular cavity **420** is depicted (when viewed down upon axis A) that can accommodate a typical luer lock fitting found on most existing luer lock syringes. Also depicted in Figure 4 is a depression **430** and female portion that when mated with the sealing conical mound **330** of soft plug portion **300** in the sealed container closure assembly **600,** will form a one way valve during patient use.

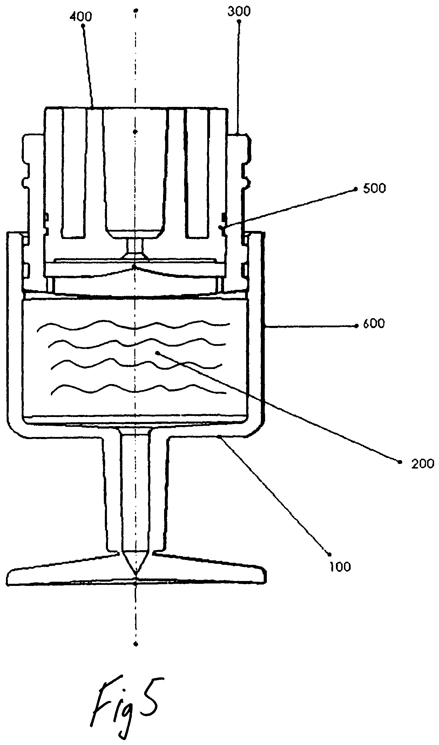

Figure 5 shows a cross sectional view of an embodiment of the container closure assembly **600** whereupon a plunger assembly **500** consisting of a soft plug portion **300** and a hard plug portion **400** are installed upon the product container **100** after the filling the product container **100** with liquid active ingredient **200** and prior to placement of the container closure assembly within a freeze drying apparatus, i.e., the plunger assembly **500** is installed in an "open" position in the product container **100.**

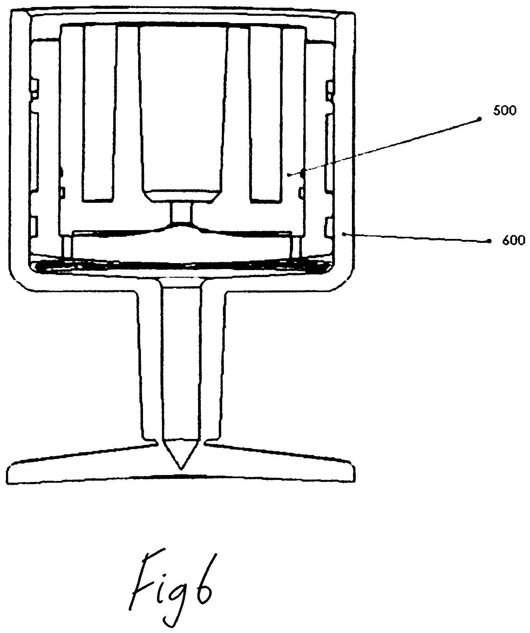

Figure 6 shows a cross sectional area of an embodiment of the container closure assembly **600** upon completion of the freeze drying cycle whereupon the liquid active ingredient has formed into a dry powder and the plunger assembly **500** has been compressed by the freeze dryer shelves to create a sealed container closure assembly **600.**

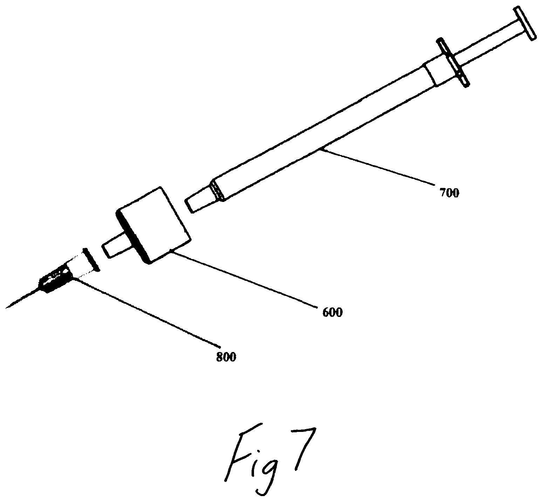

Figure 7 shows the intended use of the sealed container closure assembly **600** of the present invention with a pre-filled syringe **700** and needle **800.** In Figure 7, the base **110** attached to the neck area **120** of the assembly **600** has been broken off to allow for attachment of a needle **800.**

Contemplated for use in the container closure assembly of the present invention are storage stable powder formulations of pharmaceutical products. Importantly, the powder formulations of the present invention are optimized to produce powders which provide for "rapid" dissolution of the lyophilized powder, i.e., the powders are readily and immediately dissolved upon contact with a liquid diluent. The lyophilized powders of the present invention comprise an active ingredient, e.g., protein, and a stabilizer. Stabilizers are added to the lyophilized formulation to enhance the stability of active ingredient. Stabilizers such as, e.g., surfactants, sugars, polymers, antioxidants, amino acids, can be added to stabilize active ingredient during freezing process; and additives that can replace hydrogen bonds of water during dehydration process, e.g., sucrose, trehalose, lactose, or other sugars, can be added to stabilize pharmaceuticals by maintaining their native structure.

In order to maintain large surface area, the powder formulations may further comprise bulking agents that can form crystalline matrices (e.g., mannitol, glycine, polyethylene glycol, and the like). Alternatively, other glassy bulking agents like sugars and polymers, e.g., sucrose, trehalose, lactose, proteins, dextran and its derivatives, cyclodextran, carboxymethylcellulose, PVA, PVC, startch and its derivatives, can be added to the formulation.

The powder formulations may further comprise surfactants and buffers. Such surfactants include polysorbate 80 (or Tween 80), polysorbate 20 (or Tween 20), or pluronics. Such buffers include, e.g., phosphate, histidine, imidazole, citrace, acetate, succinate, glutamate, and glycine can be added to keep desirable pH.

In order to minimize the mass that needs to be dissolved during injection, the formulation can be composed mostly by active ingredients. For example, protein or peptide products can be lyophilized with the final solid content of 95% of protein or peptide and 5% of stabilizer.

Pharmaceutical products (active ingredients) contemplated for use include small molecules, vaccines, live or attenuated cells, oligonucleotides, DNA, peptides, and recombinant or naturally occurring proteins, whether human or animal, useful for prophylactic, therapeutic or diagnostic application. The active ingredient can be natural, synthetic, semi-synthetic or derivatives thereof. In addition, active ingredients of the present invention can be perceptible. A wide range of active ingredients are contemplated. These include but are not limited to hormones, cytokines, hematopoietic factors, growth factors, antiobesity factors, trophic factors, anti-inflammatory factors, and enzymes One skilled in the art will readily be able to adapt a desired active ingredient to the powdered formulations of present invention.

Active ingredients can include but are not limited to insulin, gastrin, prolactin, adrenocorticotropic hormone (ACTH), thyroid stimulating hormone (TSH), luteinizing hormone (LH), follicle stimulating hormone (FSH), human chorionic gonadotropin (HCG), motilin, interferons (alpha, beta, gamma), interleukins (IL-1 to IL-12), interleukin-1 receptor antagonists (IL-1ra), tumor necrosis factor (TNF), tumor necrosis factor-binding protein (TNF-bp), erythropoietin (EPO), granulocyte-colony stimulating factor (G-CSF), stem cell factor (SCF), leptin (OB protein), brain derived neurotrophic factor (BDNF), glial derived neurotrophic factor (GDNF), neurotrophic factor 3 (NT3), fibroblast growth factors (FGF), neurotrophic growth factor (NGF), bone growth factors such as osteoprotegerin (OPG), insulin-like growth factors (IGFs), macrophage colony stimulating factor (M-CSF), granulocyte macrophage colony stimulating factor (GM-CSF), megakaryocyte derived growth factor (MGDF), keratinocyte growth factor (KGF), thrombopoietin, platelet-derived growth factor (PGDF), novel erythropoiesis stimulating protein (NESP), bone morphogenetic protein (BMP), superoxide dismutase (SOD), tissue plasminogen activator (TPA), urokinase, streptokinase and kallikrein. The term proteins, as used herein, includes peptides, polypeptides, consensus molecules, analogs, derivatives or combinations thereof.

In one embodiment of the present invention, the lyophilized formulation comprises a protein drug substance, interleukin-1 receptor antagonist (IL-1ra), and standard excipients, glycine, sucrose and polysorbate 20.

Diluent to be used with the powders contained within the container closure assembly can also be customized for the best stability and patient compliance. Diluents contemplated for use include commercially available water for injection (WFI), bacteriostatic water for injection (BWFI), or phosphate buffered saline (PBS), etc. Custom developed diluent can further contain a buffering agent, e.g., acetate, phosphate, histidine, citrace, acetate, succinate, glutamate, and glycine; surfactants; stabilizers; tonicity modifiers like sodium chloride; metal ions; local anesthetic agents like lidocaine or benzyl alcohol, and hydrogels for controlled release, etc.

Materials contemplated for use in the manufacturing of the product container and the hard plug portion of the present invention include, e.g., polycarbonate, polystyrene, Teflon, and the like. Such materials are well known to those of ordinary skill in the art and readily available.

Materials contemplated for use in the manufacturing of the soft plug portion of the present invention include rubber or other pharmaceutically acceptable material that offer appropriate sealing properties. Such materials are well known to those of ordinary skill in the art and readily available.

It is understood that the container closure assembly of the present invention may vary in size and is readily adaptable to and functional with any standard type pre-filled syringe and standard type needles. Such syringes and needles are well known to those of ordinary skill in the art and readily available. Generally, the container physical dimensions should be roughly no more than 15mm x 15mm x 15mm and the container should have provisions for filling up to 1.5 ml of liquid pharmaceutical product to be lyophilized.

In the improved process for the preparation of a container closure assembly containing a lyophilized powder product, 1) the empty product container is loaded into a industry standard vial manufacturing filling line in a similar manner as regular vials; 2) the product container is filled with an optimized liquid formulation containing a pharmaceutical product; 3) the hard plug component is inserted snugly into the soft plug component to create a plunger assembly; 4) the plunger assembly is dropped into an "open" position on top of the product container, sealing the product container in the same manner as lyophilization stoppers are mounted to regular vials, creating a container closure assembly; 5) the container closure assembly is then placed into the lyophilizer and subjected to a lyophilization process; 6) during lyophilization, vapor escapes via the openings within the plunger assembly; and 7) upon completion of lyophilization, vertical compression of the lyophilizer shelves will seal the plunger assembly into the product container creating a sealed container closure assembly with minimal head space and which retains the sterility of the pharmaceutical product. Importantly, in this process, the plunger assembly is compressed such that it rests directly on top of the pharmaceutical powder and there is no air space between the powder and the plunger assembly (see Figure 6). This design concept facilitates the direct injection of the lyophilized powder without the need for a separate reconstitution/mixing/priming step of powder with diluent. In addition, the sealed container closure assembly of the present invention is able to retain the sterility of the pharmaceutical powder product and is storage stable at room temperature over the shelf life of the product.

In an improved method for the administration of a lyophilized pharmaceutical product using the container closure assembly of the present invention, 1) the sealed container closure assembly is attached at one end via friction fit to either a luer-lock or luer-slip pre-filled syringe containing the diluent; 2) the detachable base located on the neck end of the container closure assembly is removed by applying a tangential force at the base, thus exposing a luer-slip tip for the attachment of a needle; 3) a luer-slip needle is attached via friction fit to the exposed luer-slip tip of the container closure assembly; 4) the injection is then initiated by inserting the needle into the injection site; and 5) force is applied to the syringe plunger whereupon the diluent in the syringe will be forced through the plunger assembly (more specifically, the diluent will flow through Component A and into Component B via the one-way valve created by the union of Components A and B, then flow through the central channel and exit the openings in Component B); 6) the diluent will encounter the lyophilized powder in Component C and rapidly reconstitute; and 7) the reconstituted liquefied product mixture exits the container closure assembly at the luer-tip at the end of the neck area of Component C, passes through the attached needle and into the injection site. As an alternative to steps 2) and 3), the container closure assembly may have a staked needle (with a needle shield) attached at the neck end, and the needle shield removed prior to performing step 4). Importantly, the method does not require a separate reconstitution/mixing/priming step, thereby providing for a more convenient and ease of use for the patient and/or end user.

And, importantly, the improved delivery method of the present invention provides a 'gradient delivery' of the injectable pharmaceutical product. For example, because the present invention provides for the immediate reconstitution of the powdered drug upon contact with the diluent, the product is injected into the patient in a manner wherein more highly concentrated product is injected initially. It is the improved process and container closure assembly design concept described herein that facilitates the direct administration of the powdered active ingredient, without the need for a separate reconstitution/mixing step. It is thus envisioned that the lyophilized formulations, lyophilization processes and closure assembly design concepts described herein could be applied to existing delivery devices, e.g., pen systems, autoinjector systems, needle-free injector systems, dual-chambered injection cartridges and/or pre-filled syringe systems, to provide for improved methods of administration which provide for gradient delivery and which are more user friendly for the patient and/or end user.

### Example 1

In this Example, a study was conducted to demonstrate the 'gradient delivery' injection profile associated with the administration of a powdered drug using the formulations, lyophilization processes and container closure assembly design of the present invention.

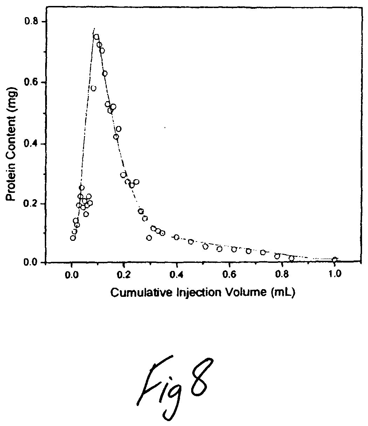

The study was performed utilizing a model protein drug substance, interleukin-1 receptor antagonist with standard excipients glycine, sucrose and polysorbate 20. The study was performed by using a sealed container closure assembly prepared using the process of the present invention and containing 10mg of IL-lra powder which was dried in a typical lyophilization process. A syringe containing 1 ml of diluent (water) was attached to the plunger assembly of the container closure assembly and the detachable base at the neck end of the container closure assembly was removed. Force is applied to the syringe plunger such that the water flows through the assembly, reconstitutes the powder, and the resultant solution drips out of the ejection port of the assembly. The concentration of IL-lra in each drop of solution was measured with a ultraviolet spectrometer. The data collected and shown in Figure 8 characterize the general profile of the gradient delivery associated with the administration of a powdered drug using the formulations, lyophilization processes and container closure assembly design of the present invention. As depicted in Figure 8, the concentration of the dose delivered over the injection volume for a gradient delivery is non constant with the bulk of the active pharmaceutical ingredient being delivered during the initial portion of the injection.

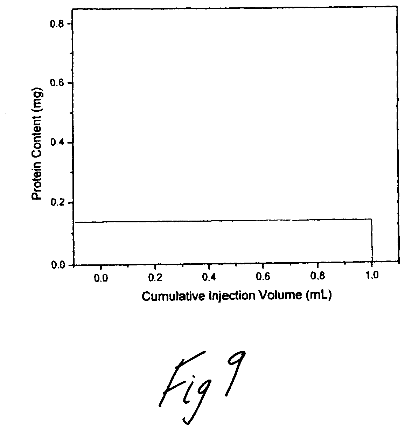

This unique gradient delivery of the injectable pharmaceutical powder product may be advantageous to the patient in certain therapeutic settings. To date, none of the known prior art delivery techniques and devices used for delivery of powdered drugs have such a profile, as all require a reconstitution and/or mixing step of the powdered drug with a diluent prior to injection, and therefore have an injection profile similar to that depicted in Figure 9. Although this specific protein was used, it is highly probable that for those skilled in the art and for most standard active pharmaceutical products, excipients and other ingredients that the same results can be achieved and will reflect these same characteristics and injection response.

The improved lyophilized formulations, lyophilization processes and closure assembly design concepts of the present invention provide patients and end-users with an alternative, less expensive and easier to use device than current state-of-the-art delivery systems for lyophilized products. Utilization of the design concept described for container closure assembly of the present invention on existing delivery devices would provide a valuable and much needed benefit to those patients dependent upon powdered drugs in their therapeutic settings.

## Claims

1. A delivery device for the administration of a powdered pharmaceutical product comprising a first component (700) and a second component (600):
the first component (700) having an ejection port at one end, a chamber holding a reservoir of diluent, and an activating means to facilitate the flow of said diluent through said ejection port; and
the second component (600) containing a lyophilized drug powder, said second component (600) capable of engaging said first component (700) at one end and engaging a standard type needle (800) at the opposing end, said second component (600) comprising a one-way valve mechanism formed as a plunger assembly (500) which upon activation of said first component (700), allows the diluent ejected from said first component (700) to flow into said second component (600) and encounter the powder in said second component (600), rapidly reconstitute and pass through said needle (800)
**characterised in that** said second component is a container closure assembly (600) comprising:
a) a hard plug component (400) comprising:
a female luer slip fitting cavity (410) to allow for friction fit of a standard type luer slip syringe;
a circular cavity (420) that can accommodate the standard type luer slip syringe (700); and
a depression and female portion (430) that can be mated with the sealing conical mound (330) of the soft plug component (300) to create a one way valve;
b) a soft plug component (300) comprising:
a hollow inside capable of accepting said hard plug component (400) to create the plunger assembly (500);
vent holes (310) which allow for vapors to escape during lyophilization processes;
sealing ridges (320) which serve to seal said soft plug component (300) against the interior wall of the product container component (100); and
a sealing conical mound (330) that can be mated with said depression and female portion (430) of said hard plug component (400) to create a one way valve; and
c) a product container component (100) comprising:
an open end having a chamber capable of holding a liquid to be lyophilized and capable of receiving said plunger assembly (500); and
an opposing neck area (120) having a detachable base (110) to allow for attachment of a standard type luer slip or luer lock syringe needle (800), and
**in that** in use there is no air space between the lyophilized drug powder and the plunger assembly (500).

2. A process for the preparation of a container closure assembly (600) containing a lyophilized pharmaceutical powder product, for use as second component in the delivery device of claim 1, comprising the steps of:
1) loading an empty product container (100) into an industry standard vial manufacturing filling line;
2) filling said product container (100) with a liquid formulation containing a pharmaceutical product;
3) dropping a plunger assembly (500), the plunger assembly (500) comprising a hard plug component (400) inserted into a soft plug component (300), into the top of said product container (100) to create a container closure assembly (600);
4) placing said container closure assembly (600) into a lyophilizer apparatus;
5) subjecting said container closure assembly (600) to a lyophilization process; and
6) completing said lyophilization process such that vertical compression of the lyophilizer shelves seals said plunger assembly (500) into said product container (100) to create a sealed container closure assembly (600) containing lyophilized pharmaceutical powder product with minimal head space.

3. The delivery device according to claim 1, wherein the product container component (100) further comprises a locking ridge (140) that prevents removal of the plunger assembly (500) when fully inserted into the product container component (100).

4. The delivery device according to claim 1 or claim 3, wherein the lyophilized drug powder comprises an active ingredient.

5. The delivery device according to claim 4, wherein the active ingredient comprises a small molecule, a vaccine, an oligonucleotide, a peptide, a protein, or a natural, synthetic, semi-synthetic, recombinant, or derivative thereof, useful for prophylactic, therapeutic or diagnostic application.

6. The delivery device according to claim 4, wherein the active ingredient comprises a hormone, a cytokine, a hematopoietic factor, a growth factor, an anti-obesity factor, a trophic factor, an anti-inflammatory factor, or an enzyme.

7. The delivery device according to claim 4, wherein the active ingredient comprises an insulin, a gastrin, a prolactin, an adrenocorticotropic hormone, a thyroid stimulating hormone, a luteinizing hormone, a follicle stimulating hormone, a human chorionic gonadotropin, a motilin, an interferon, an interleukin, an interleukin-1 receptor antagonist, a tumor necrosis factor, a tumor necrosis factor-binding protein, an erythropoietin, a granulocyte-colony stimulating factor, a stem cell factor, a leptin, a brain derived neurotrophic factor, a glial derived neurotrophic factor, a neurotrophic factor 3, a fibroblast growth factor, a neurotrophic growth factor, a bone growth factor, an insulin-like growth factor, a macrophage colony stimulating factor, a granulocyte macrophage colony stimulating factor, a megakaryocyte derived growth factor, a keratinocyte growth factor, a thrombopoietin, a platelet-derived growth factor, a novel erythropoiesis stimulating protein, a bone morphogenetic protein, a superoxide dismutase, a tissue plasminogen activator, an urokinase, a streptokinase or a kallikrein.

8. The delivery device according to any one of claims 1 or claims 3-7, wherein the lyophilized drug powder further comprises a stabilizer, a bulking agent, a surfactant and/or a buffer.

9. The delivery device according to any one of claims 1 or 3-8, wherein the plunger assembly comprises:
a hard plug component (400); and
a soft plug component (300) comprising a hollow inside capable of accepting said hard plug component (400) to create the plunger assembly (500).

10. The delivery device according to claim 9, wherein the hard plug component (400) comprises a female luer slip fitting cavity (410) to allow for friction fit of a standard type luer slip syringe (700).

11. The delivery device according to claim 9 or claim 10, wherein the soft plug component (300) comprises sealing ridges (320) which serve to seal said soft plug component (300) against the interior wall of the product container (100).

## Patentansprüche

1. Zufuhrvorrichtung zur Verabreichung eines pulverförmigen pharmazeutischen Produkts, umfassend eine erste Komponente (700) und eine zweite Komponente (600), wobei
die erste Komponente (700) eine Ausstoßöffnung an einem Ende, eine Kammer, die einen Behälter mit Verdünnungsmittel hält, und ein Aktivierungsmittel zur Erleichterung des Stroms des Verdünnungsmittels durch die Ausstoßöffnung aufweist; und
die zweite Komponente (600) ein lyophilisiertes Arzneipulver enthält, wobei die zweite Komponente (600) in der Lage ist, die erste Komponente (700) an einem Ende in Eingriff zu nehmen und eine Standardtyp-Nadel (800) am gegenüberliegenden Ende in Eingriff zu nehmen, wobei die zweite Komponente (600) einen Ein-Wege-Ventilmechanismus umfasst, der als Kolbengruppe (500) gebildet ist, welcher bei Aktivierung der ersten Komponente (700) ermöglicht, dass das von der ersten Komponente (700) ausgestoßene Verdünnungsmittel in die zweite Komponente (600) strömt und auf das Pulver in der zweiten Komponente (600) trifft, sich schnell wiederherstellt und die Nadel (800) durchläuft,
**dadurch gekennzeichnet, dass** die zweite Komponente eine Behälterverschlussgruppe (600) ist, die Folgendes umfasst:
a) eine Hartstopfenkomponente (400), umfassend:
ein Luerbuchsen-Steckloch (410), das einen Reibungssitz einer Standardtyp-Luer-Steckspritze ermöglicht;
ein kreisförmiges Loch (420), das die Standardtyp-Luer-Steckspritze (700) aufnehmen kann; und
einen Vertiefungs- und Buchsenabschnitt (430), der mit der dichtenden konischen Mündung (330) der Weichstopfenkomponente (300) zu einem Einwegventil zusammengefügt werden kann;
b) eine Weichstopfenkomponente (300), umfassend:
einen hohlen Innenraum, der in der Lage ist, die Hartstopfenkomponente (400) aufzunehmen, um die Kolbengruppe (500) zu erzeugen;
Belüftungslöcher (310), die das Entweichen von Dämpfen während der Lyophilisierungsprozesse zulassen;
Dichtungsrippen (320), die zum Abdichten der Weichstopfenkomponente (300) gegenüber der Innenwand der Produktbehälterkomponente (100) dienen; und eine dichtende konische Mündung (330), die mit dem Vertiefungs- und Buchsenabschnitt (430) der Hartstopfenkomponente (400) zu einem Einwegventil zusammengefügt werden kann; und
c) eine Produktbehälterkomponente (100), umfassend:
ein offenes Ende mit einer Kammer, die in der Lage ist, eine zu lyophilisierende Flüssigkeit zu halten, und in der Lage ist, die Kolbengruppe (500) aufzunehmen; und einen gegenüberliegenden Halsbereich (120) mit einer abnehmbaren Basis (110), die das Anbringen einer Standardtyp-Luer-Steck- oder Lyer-Verriegelungs-Spritzennadel (800) ermöglicht, und dadurch,
dass in Gebrauch kein Luftraum zwischen dem lyophilisierten Arzneipulver und der Kolbengruppe (500) ist.

2. Prozess zur Vorbereitung einer Behälterverschlussgruppe (600), enthaltend ein lyophilisiertes Arzneipulverprodukt zur Verwendung als zweite Komponente in der Zufuhrvorrichtung nach Anspruch 1, umfassend die folgenden Schritte:
1) Laden eines leeren Produktbehälters (100) in eine Befülllinie zur Herstellung von Industriestandard-Phialen;
2) Befüllen des Produktbehälters (100) mit einer flüssigen Formulierung, enthaltend ein pharmazeutisches Produkt;
3) Absenken einer Kolbengruppe (500), wobei die Kolbengruppe (500) eine in eine Weichstopfenkomponente (300) eingeführte Hartstopfenkomponente (400) umfasst, in die Oberseite des Produktbehälters (100), um eine Behälterverschlussgruppe (600) zu erzeugen;
4) Platzieren der Behälterverschlussgruppe (600) in eine Lyophilisieranordnung;
5) Unterziehen der Behälterverschlussgruppe (600) einem Lyophilisierprozess; und
6) Abschließen des Lyophilisierprozesses, so dass die vertikale Komprimierung der Lyophilisierablagen die Kolbengruppe (500) in den Produktbehälter (100) abdichtet, um eine abgedichtete Behälterverschlussgruppe (600), die lyophilisiertes pharmazeutisches Pulverprodukt mit minimalen Freiraum enthält, zu erzeugen.

3. Zufuhrvorrichtung nach Anspruch 1, wobei die Produktbehälterkomponente (100) ferner eine Verriegelungsrippe (140) umfasst, die das Entfernen der Kolbengruppe (500) verhindert, wenn diese vollständig in die Produktbehälterkomponente (100) eingeführt ist.

4. Zufuhrvorrichtung nach Anspruch 1 oder Anspruch 3, wobei das lyophilisierte Arzneimittelpulver einen Wirkstoff enthält.

5. Zufuhrvorrichtung nach Anspruch 4, wobei der Wirkstoff ein kleines Molekül, einen Impfstoff, ein Oligonukleotid, ein Peptid, ein Protein oder ein(e) natürliche(s), synthetische(s), semi-synthetische(s) Rekombinante oder Derivat davon umfasst, die/das für eine prophylaktische, therapeutische oder diagnostische Anwendung nützlich ist.

6. Zufuhrvorrichtung nach Anspruch 4, wobei der Wirkstoff ein Hormon, ein Cytokin, einen hämatopoetischen Faktor, einen Wachstumsfaktor, einen Anti-Adipositas-Faktor, einen trophischen Faktor, einen Anti-Entzündungsfaktor oder ein Enzym umfasst.

7. Zufuhrvorrichtung nach Anspruch 4, wobei der Wirkstoff ein Insulin, ein Gastrin, ein Prolactin, ein adrenokortikotropes Hormon, ein thyroidstimulierendes Hormon, ein luteinisierendes Hormon, ein follikelstimulierendes Hormon, ein humanes Choringonadotropin, ein Motilin, ein Interferon, ein Interleukin, einen Interleukin-1-Rezeptor-Antagonisten, einen Tumornekrosefaktor, ein tumornekrosefaktorbindendes Protein, ein Erythropoetin, einen granulozytkoloniestimulierenden Faktor, einen Stammzellenfaktor, ein Leptin, einen hirnabgeleiteten neurotrophischen Faktor, einen glial-abgeleiteten neurotrophischen Faktor, einen neurotrophischen Faktor 3, einen Fibroblastwachstumsfaktor, einen neurotrophischen Wachstumsfaktor, einen Knochenwachstumfasktor, einen insulinartigen Wachstumsfaktor, einen makrophagenkoloniestimulierenden Faktor, einen granulozytmakrophagenkoloniestimulierenden Faktor, einen megakaryozyt-abgeleiteten Wachstumsfaktor, einen Keratinozyt-Wachstumsfaktor, ein Thrombopoetin, einen blutplättchen-abgeleiteten Wachstumsfaktor, ein neuartiges erythropoese-stimulierendes Protein, ein knochenmorphogenetisches Protein, eine Superoxid-Dismutase, einen Gewebe-Plasminogen-Aktivator, eine Urokinase, eine Streptokinase oder ein Kallikrein umfasst.

8. Zufuhrvorrichtung nach einem der Ansprüche 1 oder Ansprüche 3-7, wobei das lyophilisierte Arzneipulver ferner einen Stabilisator, einen Füllstoff, einen oberflächenaktiven Stoff und/oder einen Puffer umfasst.

9. Zufuhrvorrichtung nach einem der Ansprüche 1 oder 3-8, wobei die Kolbengruppe umfasst:
eine Hartstopfenkomponente (400); und
eine Weichstopfenkomponente (300), umfassend einen hohlen Innenraum, der in der Lage ist, die Hartstopfenkomponente (400) aufzunehmen, um die Kolbengruppe (500) zu erzeugen.

10. Zufuhrvorrichtung nach Anspruch 9, wobei die Hartstopfenkomponente (400) ein Luerbuchsen-Steckloch (410), das einen Reibungssitz einer Standardtyp-Luer-Steckspritze (700) ermöglicht, umfasst.

11. Zufuhrvorrichtung nach Anspruch 9 oder Anspruch 10, wobei die Weichstopfenkomponente (300) Dichtungsrippen (320) umfasst, die zum Abdichten der Weichstopfenkomponente (300) gegenüber der Innenwand des Produktbehälters (100) dienen.

## Revendications

1. Dispositif de distribution pour l'administration d'un produit pharmaceutique en poudre comprenant un premier composant (700) et un second composant (600),
le premier composant (700) possédant un orifice d'éjection au niveau d'une extrémité, une chambre abritant un réservoir de diluent et un moyen d'actionnement pour faciliter l'écoulement dudit diluent à travers ledit orifice d'éjection ; et
le second composant (600) contenant une poudre de médicament lyophilisée, ledit second composant (600) étant capable de se mettre en prise avec ledit premier composant (700) au niveau d'une extrémité et de se mettre en prise avec une aiguille de type standard (800) au niveau de l'extrémité opposée, ledit second composant (600) comprenant un mécanisme de soupape une voie formé sous la forme d'un ensemble piston plongeur (500) qui lors de l'actionnement dudit premier composant (700) permet au diluent éjecté dudit premier composant (700) de s'écouler dans ledit second composant (600) et de rencontrer la poudre dans ledit second composant (600), de se reconstituer rapidement et de passer à travers ladite aiguille (800)
**caractérisé en ce que** ledit second composant est un ensemble de fermeture de contenant (600) comprenant :
a) un composant de bouchon dur (400) comprenant :
une cavité de raccord à glissement luer femelle (410) permettant un ajustement par friction d'une seringue à glissement luer de type standard ;
une cavité circulaire (420) pouvant accueillir la seringue à glissement luer de type standard (700) ; et
une partie de dépression et femelle (430) pouvant s'accoupler avec la partie en forme de monticule conique étanche (330) du composant de bouchon souple (300) pour créer une soupape une voie ;
b) un composant bouchon souple (300) comprenant :
un intérieur creux capable d'accepter ledit composant bouchon dur (400) pour créer l'ensemble piston plongeur (500) ;
des trous de ventilation (310) permettant aux vapeurs de s'évacuer durant le processus de lyophilisation ;
des nervures d'étanchéité (320) qui servent à assurer l'étanchéité dudit composant bouchon souple (300) contre la paroi intérieure du composant de contenant de produit (100) ; et une partie en forme de monticule conique d'étanchéité (330) qui peut être accouplée à ladite partie de dépression et femelle (430) du composant bouchon dur (400) pour créer une soupape une voie ; et
c) un composant de contenant de produit (100) comprenant :
une extrémité ouverte possédant une chambre capable de contenir un liquide à lyophiliser et capable de recevoir ledit ensemble piston plongeur (500) ; et une zone de goulot opposée (120) possédant une base détachable (110) pour permettre la fixation d'une aiguille de seringue à coulissement luer ou à verrouillage luer de type standard (800), et
**en ce que** lors de l'utilisation aucun espace d'air n'est présent entre la poudre de médicament lyophilisée et l'ensemble piston plongeur (500).

2. Procédé permettant la préparation d'un ensemble de fermeture de contenant (600) contenant un produit en poudre pharmaceutique lyophilisé, destiné à être utilisé en tant que second composant dans le dispositif de distribution selon la revendication 1, comprenant les étapes de :
1) chargement d'un contenant de produit vide (100) dans une ligne de remplissage de fabrication de flacon standard du commerce ;
2) remplissage dudit contenant de produit (100) avec une formulation liquide contenant un produit pharmaceutique ;
3) descente d'un ensemble piston plongeur (500), ledit ensemble piston plongeur (500) comprenant un composant bouchon dur (400) inséré dans un composant bouchon souple (300), dans le haut dudit contenant de produit (100) pour créer un ensemble de fermeture de contenant (600) ;
4) mise en place dudit ensemble de fermeture de contenant (600) dans un appareil lyophilisateur ;
5) soumission dudit ensemble de fermeture de contenant (600) à un processus de lyophilisation ; et
6) réalisation dudit processus de lyophilisation de sorte qu'une compression verticale des étagères du lyophilisateur scelle ledit ensemble piston plongeur (500) dans ledit contenant de produit (100) pour créer un ensemble de fermeture de contenant scellé (600) contenant le produit en poudre pharmaceutique lyophilisé avec un espace de tête minimal.

3. Dispositif de distribution selon la revendication 1, ledit composant de contenant de produit (100) comprenant en outre une nervure de blocage (140) qui empêche le retrait de l'ensemble piston plongeur (500) lorsqu'il est complètement inséré dans le composant de contenant de produit (100).

4. Dispositif de distribution selon la revendication 1 ou 3, ladite poudre de médicament lyophilisée comprenant un ingrédient actif.

5. Dispositif de distribution selon la revendication 4, ledit ingrédient actif comprenant une petite molécule, un vaccin, un oligonucléotide, un peptide, une protéine, ou un recombinant ou un dérivé naturelle, synthétique ou semi-synthétique de ceux-ci, utile pour une application prophylactique, thérapeutique ou diagnostique.

6. Dispositif de distribution selon la revendication 4, ledit ingrédient actif comprenant une hormone, une cytokine, un facteur hématopoïétique, un facteur de croissance, un facteur de lutte contre l'obésité, un facteur trophique, un facteur d'anti-inflammatoire ou une enzyme.

7. Dispositif de distribution selon la revendication 4, ledit ingrédient actif comprenant une insuline, gastrine, une prolactine, une hormone adrénocorticotrope, une hormone stimulant la thyroïde, une hormone lutéinisante, une hormone folliculostimulante, une gonadotrophine chorionique humaine, une motiline, un interféron, une interleukine, un antagoniste de récepteur d'interleukine-1, un facteur de nécrose tumorale, une protéine de fixation sur le facteur de nécrose tumorale, une érythropoïétine, un facteur de stimulation des colonies de granulocytes, un facteur des cellules souches, une leptine, un facteur neurotrophique dérivé du cerveau, un facteur neurotrophique dérivé des cellules gliales, un facteur neurotrophique 3, un facteur de croissance fibroblastique, un facteur de croissance neurotrophique, un facteur de croissance des os, un facteur de croissance analogue à l'insuline, un facteur de stimulation de colonies de macrophages, un facteur de stimulation de colonies de granulocytes et de macrophages, un facteur de croissance dérivé de mégacaryocyte, un facteur de croissance des kératinocyte, une thrombopoïétine, un facteur de croissance dérivé des plaquettes, une protéine nouvelle de stimulation de l'érythropoïèse, une protéine morphogénétique osseuse, une superoxyde dismutase, un activateur tissulaire du plasminogène, une urokinase, une streptokinase ou une kallicréine.

8. Dispositif de distribution selon l'une quelconque des revendications 1 ou des revendications 3 à 7, ladite poudre de médicament lyophilisé comprenant en outre un stabilisant, un agent gonflant, un tensioactif et/ou un tampon.

9. Dispositif de distribution selon l'une quelconque des revendications 1 ou 3 à 8, ledit ensemble piston plongeur comprenant :
un composant bouchon dur (400) ; et
un composant bouchon souple (300) comprenant un intérieur creux capable d'accepter ledit composant bouchon dur (400) pour créer l'ensemble piston plongeur (500).

10. Dispositif de distribution selon la revendication 9, ledit composant bouchon dur (400) comprenant une cavité de raccord à glissement luer femelle (410) permettant un ajustement par friction d'une seringue à glissement luer de type standard (700).

11. Dispositif de distribution selon la revendication 9 ou 10, ledit composant bouchon souple (300) comprenant des nervures d'étanchéité (320) servant à sceller ledit composant bouchon souple (300) contre la paroi intérieure du contenant de produit (100).
